(19) Europäisches Patentamt
European Patent Office
Office européen des brevets

(11) **EP 2 988 224 A1**

(12) **DEMANDE DE BREVET EUROPEEN**

(43) Date de publication:
**24.02.2016 Bulletin 2016/08**

(51) Int Cl.:
***G06F 17/18*** (2006.01)  ***G06F 3/01*** (2006.01)
***A61B 5/04*** (2006.01)

(21) Numéro de dépôt: **15181681.6**

(22) Date de dépôt: **20.08.2015**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA**

(30) Priorité: **22.08.2014 FR 1457947**

(71) Demandeur: **Commissariat à l'Énergie Atomique et aux Énergies Alternatives**
**75015 Paris (FR)**

(72) Inventeurs:
• **LABYT, Etienne**
  **38950 SAINT MARTIN LE VINOUX (FR)**
• **DURAND, Pierre**
  **38700 Corenc (FR)**

(74) Mandataire: **Brevalex**
**56, Boulevard de l'Embouchure**
**B.P. 27519**
**31075 Toulouse Cedex 2 (FR)**

(54) **PROCÉDÉ DE LOCALISATION D'UNE ACTIVITÉ CÉRÉBRALE ASSOCIÉE À UNE TÂCHE**

(57) L'invention concerne une méthode d'estimation de l'activité cérébrale, à partir de signaux physiologiques, en particulier de signaux magnétoencéphalographiques ou électroencéphalographiques, qui présente, dans certaines zones prédéterminées du cortex, considérées comme des zones d'intérêt, une précision améliorée par rapport à d'autres zones du maillage. Cela permet d'obtenir une estimation plus précise dans les zones du cerveau destinées à faire l'objet d'un traitement particulier, par exemple accueillir des électrodes corticales.

**Fig. 1**

**EP 2 988 224 A1**

**Description**

**DOMAINE TECHNIQUE**

La présente invention concerne de manière générale l'estimation de l'activité électrique dans un tissu humain ou animal, et plus particulièrement la localisation d'une activité cérébrale à partir de signaux physiologiques, obtenus par magnétoencéphalographie ou par électroencéphalographie. L'invention s'applique notamment au domaine de la neuroimagerie fonctionnelle et de la commande neuronale directe.

**ÉTAT DE LA TECHNIQUE ANTÉRIEURE**

Les méthodes de neuroimagerie fonctionnelle se divisent classiquement entre celles basées sur l'activité métabolique telles que l'imagerie par résonance magnétique fonctionnelle (IRMf), représentant la réponse hémodynamique, ou la tomographie par émission de positrons (TEP), représentant les modifications du débit sanguin, et les méthodes basées sur l'activité électrophysiologique, telles que l'électroencéphalographie (EEG), mesurant l'activité électrique cérébrale au moyen d'électrodes placées sur le scalp du sujet, l'électrocorticographie (ECoG) mesurant l'activité cérébrale au moyen d'électrodes placées directement sur la surface corticale, ou encore la magnétoencéphalographie (MEG) mesurant les champs magnétiques liés à l'activité électrique dans le cerveau.

Les méthodes d'imagerie fonctionnelle EEG et MEG présentent une meilleure résolution temporelle que les méthodes IRMf et TEP. En outre, elles sont non invasives contrairement à l'électrocorticographie. Enfin, la magnétoencéphalographie est particulièrement intéressante en ce que les signaux magnétiques engendrés par les courants dans le cerveau (principalement les courants ioniques dans les dendrites lors de la transmission synaptique), ne subissent pas ou peu de distorsion lorsqu'ils se propagent au travers de la boîte crânienne.

Par ailleurs, l'électroencéphalographie et la magnétoencéphalographie font actuellement l'objet de recherches importantes pour leurs applications potentielles à la commande neuronale directe. La commande neuronale directe ou BCI (*Brain Computer Interface*) permet d'établir une communication directe entre le cerveau d'un utilisateur et un dispositif externe (ordinateur, système électronique, effecteur) sans médiation par une voie musculaire. La commande neuronale directe exploite l'association d'une ou de plusieurs tâches mentales (actions imaginées par le sujet) à une ou plusieurs commandes du dispositif externe. Ainsi une action de la main imaginée par le sujet pourra être associée à un déplacement d'un curseur sur l'écran d'un ordinateur ou le mouvement d'un effecteur. Cette technique est très prometteuse notamment pour des personnes atteintes de paralysie.

Que ce soit dans le domaine de la neuroimagerie fonctionnelle ou dans celui de la commande directe, différentes méthodes ont été mises au point pour localiser une activité cérébrale associée à une tâche (imaginée ou effectuée) à partir de signaux physiologiques acquis par une pluralité de capteurs. Ainsi, dans le cas de l'EEG, les capteurs sont des électrodes permettant d'acquérir des différences de potentiel électriques à la surface du scalp. Dans le cas de la MEG les capteurs (SQUIDs), peuvent être des magnétomètres capables de mesurer l'intensité du champ magnétiques et/ou des gradiomètres planaires (ou axiaux) capables de mesurer le gradient du champ magnétique dans un plan donné. Par exemple, un équipement MEG peut combiner, en une même localisation, trois capteurs élémentaires voire plus : un magnétomètre de précision mesurant l'intensité et l'orientation du champ magnétique en un point et deux gradiomètres planaires, perpendiculaires entre eux mesurant deux composantes du gradient du champ magnétique en ce point.

En tout état de cause, les méthodes précitées ont pour objet de localiser les sources d'activité cérébrale à partir des signaux acquis par les différents capteurs. Plus précisément, si l'on effectue un maillage du cortex en $M$ zones élémentaires et si l'on note **x** un vecteur (de taille $M$) représentatif des densités de courant électrique (ou signaux source) dans les différentes zones élémentaires et **y** un vecteur (de taille $N$) représentatif des signaux acquis par les différents capteurs, on a la relation matricielle :

$$\mathbf{y} = \mathbf{A}\mathbf{x} + \mathbf{b} \tag{1}$$

où **A** est une matrice de taille $N \times M$, dite matrice de transmission (*lead field matrix*) fonction de la zone élémentaire considérée et **b** est un vecteur d'échantillons de bruit de taille $N$.

Ces $M$ zones élémentaires sont réparties à la surface du Cortex, selon un maillage prédéterminé. On estime alors le signal neuronal en $M$ points représentatifs de ces zones élémentaires. Ces $M$ points sont déterminés en fonction du maillage. Il peut s'agir du centre ou des sommets de chaque maille.

La matrice **A** est généralement obtenue par simulation à partir d'une modélisation du cerveau par éléments frontières ou finis. D'une façon générale, chaque terme A(i,j) de la matrice A représente le signal mesuré i correspondant à une activité électrique unitaire dans la zone élémentaire j.

Ainsi, le cortex est divisé en *M* zones élémentaires et chaque élément du vecteur **x** étant représentatif de la densité de courant électrique dans un voxel, par exemple pris égal à la norme d'un vecteur de densité de courant électrique dans le voxel en question.

La localisation de l'activité cérébrale revient à rechercher le vecteur **x** (ou, à tout le moins un vecteur **x**, dans la mesure où le système (1) est sous-déterminé), à partir du vecteur **y**, c'est-à-dire à inverser la relation (1). Pour cette raison, la localisation de l'activité cérébrale à partir du vecteur de signaux **y** est quelquefois appelée « problème inverse » dans la littérature. Cette inversion est délicate car il existe en fait une infinité de solutions en raison de la sous-détermination du système (1), le nombre de sources étant bien supérieur au nombre de capteurs. On est alors conduit à faire des hypothèses supplémentaires pour pouvoir réaliser l'inversion.

Différentes solutions du problème inverse ont été proposées dans la littérature, notamment la méthode MNE (*Minimum Norm Estimate*), la méthode dSPM (*dynamic Statistical Parameter Mapping*) utilisée dans la localisation de sources profondes, la méthode LORETA (*Low Resolution Electromagnetic Tomography*), les méthodes par formation de faisceau (*Beamforming*) décrites notamment dans l'article de A. Fuchs intitulé « Beamforming and its application to brain connectivity ». publié dans l'ouvrage « Handbook of Brain Connectivity », V.K. Jirsa, R.A. McIntosh, Springer Verlag, Berlin, pp. 357-378 (2007).

On trouvera par ailleurs une revue des différentes méthodes de localisation précitées dans l'article de O. Hauk et al. intitulé « Comparison of noise-normalized minimum norm estimates of MEG analysis using multiple resolution metrics » parue dans Neuroimage, Vol. 54, 2011, pp. 1966-1974.

Si l'on fait l'hypothèse que le bruit est gaussien et plus précisément que les échantillons de bruit sont des variables aléatoires gaussiennes centrées indépendantes et identiquement distribuées, la solution de (1) peut être donnée par la matrice **W** qui minimise l'erreur quadratique :

$$e = \left\| \mathbf{W}\mathbf{y} - \mathbf{x} \right\|^2 \tag{2}$$

L'équation (2) peut encore s'écrire sous la forme :

$$e = \left\| \mathbf{M}\mathbf{x} \right\|^2 + \left\| \mathbf{W}\mathbf{b} \right\|^2 = Tr\left( \mathbf{M}\mathbf{R}\mathbf{M}^T \right) + Tr\left( \mathbf{W}\mathbf{C}\mathbf{W}^T \right) \tag{3}$$

où **M** = **WA**-**I**$_M$, **I**$_M$ est la matrice identité de taille $M \times M$, *Tr* est la fonction trace, **R** est la matrice de covariance des signaux source, et **C** est la matrice de covariance du bruit. La minimisation de l'erreur quadratique *e* conduit à la solution :

$$\mathbf{W} = \mathbf{R}\mathbf{A}^T \left( \mathbf{A}\mathbf{R}\mathbf{A}^T + \mathbf{C} \right)^{-1} \tag{4}$$

et donc à une estimation de la localisation de l'activité cérébrale donnée par :

$$\hat{\mathbf{x}} = \mathbf{R}\mathbf{A}^T \left( \mathbf{A}\mathbf{R}\mathbf{A}^T + \mathbf{C} \right)^{-1} \mathbf{y} \tag{5}$$

La matrice de covariance de bruit peut s'écrire **C** = $\sigma^2 \mathbf{I}_N$ où $\sigma^2$ est la variance du bruit et **I**$_N$ est la matrice unité de taille $N \times N$. De même, si les différentes sources sont considérées comme indépendantes et identiquement distribuées (même puissance pour tous les dipôles), on a **R** = $p.\mathbf{I}_M$ où *p* est la puissance du signal source dans toutes les zones élémentaires, la relation (5) peut se simplifier :

$$\hat{\mathbf{x}} = \mathbf{A}^T \left( \mathbf{A}\mathbf{A}^T + \lambda \mathbf{I}_N \right)^{-1} \mathbf{y} \tag{6}$$

où $\lambda = \sigma^2/p$ est un paramètre de régularisation traduisant l'importance du bruit sur le signal source. L'expression (6) est la solution de la méthode MNE mentionnée plus haut.

Quelle que soit la méthode de localisation envisagée, la précision de localisation obtenue décroit rapidement avec le rapport signal sur bruit ($\lambda^{-1}$). Pour pallier cette difficulté, il a été notamment proposé dans l'article de Liu et al. intitulé « Spatiotemporal imaging of human brain activity using functional MRI constrained magnetoencephalography data : Monte Carlo simulations » publié dans Proceedings of the National Academy of Sciences of the United States of America, vol. 95, n° 15, pp. 8945-8950, Juillet 1998, d'exploiter des données de localisation d'IRMf pour améliorer la précision de localisation de l'activité cérébrale MEG ou EEG. Toutefois, cette amélioration de la précision par hybridation de données suppose, d'une part, de disposer d'IRMf pour la même tâche, ce qui n'est pas envisageable pour la commande neuronale directe, et, d'autre part, que l'activité électrique/magnétique dans le cerveau est corrélée avec la réponse hémodynamique. De ce fait, elle introduit inévitablement un biais en orientant la localisation de l'activité cérébrale MEG ou EEG vers des sources qui ont été détectées par IRMf.

Le problème à la base de la présente invention est par conséquent de proposer une méthode d'estimation de l'activité cérébrale associée, à partir de signaux physiologiques, en particulier de signaux magnétoencéphalographiques ou électroencéphalographiques, qui présente, dans certaines zones prédéterminées du cortex, considérées comme des zones d'intérêt, une précision améliorée par rapport à d'autres zones du maillage. Cela permet d'obtenir une estimation plus précise de l'activité neuronale, et son évolution temporelle, dans les zones du cerveau destinées à faire l'objet d'un traitement particulier, par exemple accueillir des électrodes corticales.

## EXPOSÉ DE L'INVENTION

La présente invention est définie par une méthode d'estimation de l'activité électrique au sein d'un tissu d'un sujet, dans laquelle

- on décompose le tissu en une pluralité de zones élémentaires et on détermine une matrice de transition reliant l'activité électrique en chaque zone à un signal physiologique autour du tissu,
- on réalise l'acquisition d'une pluralité de signaux physiologiques grâce à une pluralité de capteurs disposés autour du tissu,
- on définit des zones élémentaires d'intérêt parmi les zones élémentaires, le nombre de zones élémentaires d'intérêt étant strictement inférieur au nombre de zones élémentaires
- on établit une matrice de covariance de sources, de telle sorte que les termes de sa diagonale, correspondant aux zones élémentaires d'intérêt, soient inférieurs aux autres termes de sa diagonale,
- on estime l'activité électrique, en au moins une zone élémentaire, à partir des signaux physiologiques, de la matrice de covariance de sources et de la matrice de covariance de bruit.

Chaque zone élémentaire d'intérêt peut correspondre à une position au niveau de laquelle une électrode corticale est destinée à être apposée, le procédé permettant alors d'estimer l'activité électrique susceptible d'être mesurée par ladite électrode, ainsi que son évolution temporelle.

Les signaux physiologiques peuvent être mesurés par une méthode de mesure non invasive, par exemple par magnétoencéphalographie ou par électroencéphalographie.

L'estimation peut être basée sur un critère MNE.

La matrice de covariance de bruit peut être avantageusement calculée comme la matrice de covariance des signaux physiologiques sur une fenêtre temporelle dans laquelle le sujet est au repos.

L'activité électrique peut alors être estimée par la relation $\hat{x} = \tilde{R} A^T (A \tilde{R} A^T + C)^{-1} y$ où $\hat{x}$ est un vecteur représentant l'activité électrique dans les différentes zones élémentaires, $y$ est un vecteur représentant les signaux physiologiques acquis par les capteurs, $A$ est une matrice estimant le signal mesuré à chaque capteur pour des sources de puissance unitaire situées dans chaque zones élémentaires, $C$ est la matrice de covariance de bruit et $\tilde{R}$ est la matrice de covariance de sources.

La matrice $\tilde{R}$ de covariance de sources peut être établie de la façon suivante :

- $\tilde{R}(i,j) = 0$ si $i \neq j$, chaque source élémentaire étant considérée comme indépendante l'une de l'autre,
- $\tilde{R}(i,i) = \lambda'$ lorsque la zone élémentaire i est une zone élémentaire d'intérêt
- $R(i,i) = \lambda$ lorsque la zone élémentaire i n'est pas une zone élémentaire d'intérêt,

$\lambda$ et $\lambda'$ étant des réels strictement positifs, avec $\lambda' < \lambda$

Il n'est pas nécessaire que tous les termes de la diagonale de la matrice de covariance de sources, correspondant aux zones élémentaires d'intérêt aient la même valeur $\lambda'$. De manière similaire, il n'est pas nécessaire que tous les termes de la diagonale, correspondant aux zones élémentaires qui ne sont pas des zones élémentaires d'intérêt est la même valeur $\lambda$. La matrice de covariance de sources $\tilde{R}$ peut dans ce cas être établie de la façon suivante :

- $\tilde{R}(i,j) = \lambda_{ij}$ si $i \neq j$, les source élémentaires distinctes étant considérées comme indépendante sl'une de l'autre, $\lambda_{ij}$ pouvant être, par exemple, égal à 0 pour l'ensemble des coefficients $\tilde{R}(i,j)$ avec $i \neq j$.
- $\tilde{R}(i,i) \lambda_i'$ lorsque la zone élémentaire i est une zone élémentaire d'intérêt, la valeur $\lambda_i'$ dépendant de l'indice i

- $R(i,i) = \lambda_i$ lorsque la zone élémentaire i n'est pas une zone élémentaire d'intérêt, la valeur $\lambda_i$ dépendant de l'indice i

Chaque terme $\lambda_i$ ou $\lambda_i'$ étant des réels strictement positifs associés à la zone élémentaire i, chaque terme $\lambda_i'$ associé à une zone élémentaire d'intérêt étant inférieur à aux termes $\lambda_i$ associés aux autres zones élémentaires. On établit ainsi une hiérarchie dans la zone élémentaire d'intérêt et dans les zones élémentaires en dehors de la zone élémentaire d'intérêt, cette hiérarchie étant gouvernée respectivement par $\lambda_i'$ ou $\lambda_i$.

Selon un second mode de réalisation, l'activité électrique est associée à une tâche, effectuée, imaginée ou réalisée par le sujet lorsque celui-ci reçoit un stimulus, l'activité électrique étant alors mesurée dans une période temporelle suivant ledit stimulus, la méthode comportant alors les étapes suivantes :

- on calcule des coefficients de corrélation entre les différents signaux physiologiques et un signal représentatif dudit stimulus
- on pondère les coefficients de la matrice de covariance, à l'aide des coefficients de corrélation, de sorte à pénaliser, en termes de rapport signal sur bruit, les signaux physiologiques qui sont faiblement corrélés avec le stimulus, la pénalisation diminuant les termes de la matrice de corrélation relatifs aux signaux faiblement corrélés avec le stimulus.

En particulier, l'estimation est basée sur un critère de type MNE, et, de préférence, la matrice de covariance des signaux physiologiques est une matrice de covariance de bruit, calculée sur une fenêtre temporelle où le stimulus est absent, l'activité électrique dans chaque zone élémentaire étant alors estimée selon l'expression : $\hat{x}(t) = \tilde{R}A^T(A\tilde{R}A^T + \tilde{C}(t))^{-1}y(t)$ où $\hat{x}(t)$ est un vecteur représentant l'activité électrique au temps t dans les différentes zones élémentaires, $y(t)$ est un vecteur représentant les signaux physiologiques acquis par les capteurs au temps t, **A** est une matrice estimant le signal mesuré à chaque capteur pour des sources de puissance unitaire situées dans chaque zones élémentaires, $\tilde{C}(t)$ est la matrice de covariance de bruit au temps t et $\tilde{R}$ est la matrice de covariance de sources.

Avantageusement, les coefficients de matrice de covariance de bruit pondérée sont obtenus à partir de la matrice de covariance de bruit au moyen de la relation suivante :

$$\tilde{C}_{ij}(t) = C_{ij} \text{ pour } i = 1,...,N, j = 1,...,N, i \neq j$$

$$\tilde{C}_{ii}(t) = \frac{C_{ii}}{1 + \gamma^2 \chi_i^2(t)} \text{ pour } i = 1,...,N$$

où les coefficients $\tilde{C}_{ij}(t)$, $i=1,...,N,j=1,...,N$, sont les coefficients de la matrice de covariance de bruit pondérée, les coefficients $C_{ij}$, $i=1,...,N,j=1,...,N$ sont les coefficients de la matrice de covariance de bruit, $N$ est le nombre de capteurs, $\gamma$ est une constante réelle prédéterminé et $\chi_i(t)$, $i=1,...,N$, sont les coefficients de corrélation des signaux physiologiques acquis par les différents capteurs avec le signal représentatif du stimulus.

Les coefficients de corrélation $\chi_i(t)$, $i=1,...,N$ peuvent faire l'objet d'une normalisation préalablement à la pondération des coefficients de la matrice de covariance de bruit.

Les coefficients de corrélation peuvent être obtenus en effectuant une transformée temps-fréquence ou temps-échelle de chaque signal physiologique pour obtenir une pluralité de composantes fréquentielles ($Y_f(t)$) de ce signal en fonction du temps, en calculant les coefficients de Pearson ($R_f(t)$) entre lesdites composantes fréquentielles et le signal représentatif du stimulus, le coefficient de corrélation ($\chi(t)$) relatif à un signal physiologique étant déterminé à partir desdits coefficients de Pearson obtenus pour ce signal.

Le coefficient de corrélation ($\chi(t)$) relatif à un signal physiologique peut alors être obtenu comme la valeur extrémale des coefficients de Pearson pour les différentes composantes fréquentielles de ce signal.

Un objet de l'invention est également un procédé de définition la position d'au moins une électrode corticale, le procédé comportant :

- l'estimation de l'activité électrique, telle que définie ci-dessus, en considérant, notamment successivement, différents jeux de zones élémentaires d'intérêt, chaque jeu de zone élémentaire d'intérêt comportant au moins une zone élémentaire d'intérêt, de manière à obtenir une estimation de l'activité électrique correspondant à chaque jeu de zones élémentaires considéré,
- la comparaison de l'estimation de l'activité électrique pour chacun des différents jeux de zones élémentaires d'intérêt,
- la définition de la position optimale de chaque électrode corticale en fonction de cette comparaison.

La comparaison vise notamment à déterminer la position optimum de chaque électrode du point de vue du rapport signal sur bruit.

**BRÈVE DESCRIPTION DES DESSINS**

D'autres caractéristiques et avantages de l'invention apparaîtront à la lecture d'un mode de réalisation préférentiel de l'invention fait en référence aux figures jointes parmi lesquelles :

La Fig. 1 représente de manière schématique l'ordinogramme d'un procédé d'estimation de l'activité électrique dans un tissu selon un premier mode de réalisation de l'invention ;
La Fig. 2 représente de manière schématique l'ordinogramme d'un procédé d'estimation de l'activité électrique dans un tissu selon un second mode de réalisation de l'invention ;
La Fig. 3 représente de manière schématique un exemple de calcul d'un coefficient de corrélation entre un signal physiologique et un signal de stimulus ;

**EXPOSÉ DÉTAILLÉ DE MODES DE RÉALISATION PARTICULIERS**

On considérera par la suite un système d'acquisition de signaux physiologiques à partir d'une pluralité de capteurs disposés autour d'un tissu d'intérêt d'un sujet, humain ou animal, ce tissu étant le siège d'une activité électrique lorsque ce sujet effectue, voit ou imagine visuellement une tâche. Dans un souci d'illustration et sans préjudice de généralisation nous envisagerons plus particulièrement le cas d'un système d'acquisition magnétoencéphalographique, étant bien entendu que d'autres systèmes d'acquisition pourront être alternativement utilisés, notamment un système d'acquisition électroencéphalographique.

Le système magnétoencéphalographique comprend, de manière connue, un « casque MEG » placé à quelques centimètres de la boite crânienne du sujet. Ce casque comprend une pluralité de capteurs situés en différents points. , chaque capteur pouvant être constitué d'un ou de plusieurs capteurs élémentaires. Les capteurs élémentaires peuvent être des magnétomètres de précision et des gradiomètres planaires (ou axiaux). Alternativement, ils peuvent être des gradiomètres radiaux tels que ceux décrits dans l'article de J. Vrba et al. intitulé « Signal processing in magnetoencephalography », Methods 25, 249-271 (2001). Les mouvements de la tête du sujet sont en outre enregistrés et compensés grâce à des bobines placées en des points fixes par rapport à la tête du sujet et générant un champ magnétique dans une bande de fréquence éloignée de celle du signal MEG.

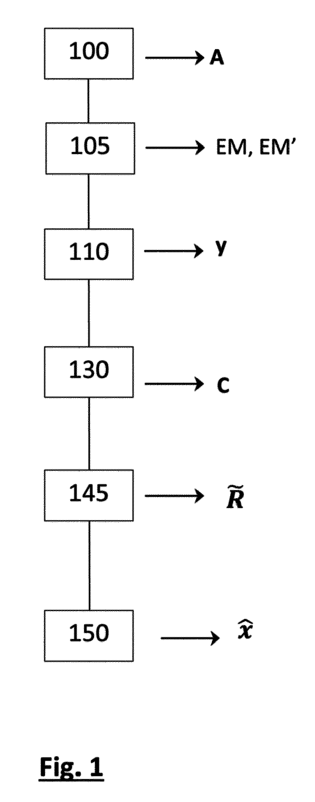

La Fig. 1 représente de manière schématique une méthode d'estimation de l'activité électrique au sein d'un tissu d'un sujet, selon un premier mode de réalisation de l'invention.

A l'étape 100, on définit, à l'aide du maillage, $M$ zones élémentaires sur le tissu observé, chaque zone élémentaire étant représentée par un point $P_m$, avec $1 \leq m \leq M$. Comme précédemment évoqué, ce point peut être le centre ou un sommet d'une maille. On détermine alors la matrice de transmission **A**, classiquement dénommée matrice de gain ou « *leadfield matrix* » (dimension $N \times M$) issue de la résolution du problème direct, dont chaque élément $A_{ij}$ représente le signal mesuré par le capteur i correspondant à une activité électrique unitaire dans la zone élémentaire j (ou au point Pj associé à la zone élémentaire j). La matrice de transmission **A** est obtenue par simulation, par exemple préalablement à l'acquisition des signaux physiologiques, en estimant les signaux mesurés par les différents capteurs pour une valeur donnée d'une source élémentaire. Le processus est répété pour les $M$ zones élémentaires de sorte que l'on obtient successivement les $M$ lignes de la matrice **A**.

A l'étape 105, on sélectionne, parmi les $M$ zones élémentaires, $M'$ zones élémentaires d'intérêt, $M'$ étant un entier positif strictement inférieur à $M$. Chaque zone élémentaire d'intérêt correspond à une zone dans laquelle on souhaite estimer l'activité neuronale avec une précision accrue, par rapport aux autres zones élémentaires $M$.

Chaque zone élémentaire d'intérêt peut correspondre à l'emplacement prévu d'une électrode corticale. La surface corticale est par exemple obtenue par une modalité IRM, auquel cas le maillage peut être réalisé dans un repère associé aux données IRM. La coordonnée de chaque électrode corticale est alors déterminée dans ce repère, et on détermine, pour chaque électrode, la zone élémentaire d'intérêt la plus proche. Cela revient à déterminer, pour chaque électrode, le point du maillage $P_m$ le plus proche, ce point étant alors considéré comme un point d'intérêt.

Une zone élémentaire d'intérêt peut également correspondre à une zone pathologique ou lésionnelle, où à une zone fonctionnelle identifiée par une modalité de type IRM fonctionnelle ou PET Scan.

Le nombre $M'$ de zones élémentaires d'intérêt est typiquement de quelques dizaines, et par exemple compris entre 5 et 100, tandis que le nombre M de zones élémentaires considérées est supérieur à 1000, voire supérieur à 10000. Finalement, cette étape revient à constituer un sous-ensemble EM' de $M'$ zones élémentaires dans l'ensemble EM regroupant les $M$ zones élémentaires du maillage.

A l'étape 110, on acquiert les signaux physiologiques à l'aide de capteurs disposés à la périphérie du cortex. Comme précédemment évoqué, ces capteurs peuvent être des électrodes, des magnétomètres ou des gradiomètres.

De préférence, ces capteurs ne sont pas invasifs et sont disposés à l'extérieur du corps. Il peut notamment s'agir d'électrodes EEG ou de capteurs magnétiques de type MEG. L'enregistrement peut avoir lieu lorsque le sujet est au repos, ou lorsque le sujet effectue, imagine ou visualise une tâche. L'enregistrement peut également avoir lieu lorsque le sujet est atteint d'une pathologie neuronale particulière, par exemple en relation avec des évènements paroxystiques intercritiques chez le patient épileptique, ou chez un patient atteint d'une maladie neuronale dégénérative ou d'une lésion traumatique ou tumorale. Lors de cette étape 110, on constitue un vecteur **y**, de taille $N$ représentatif des signaux acquis par les différents capteurs.

A l'étape 130, on calcule la matrice de covariance **C** de bruit, chaque élément $C_{ij}$ de cette matrice étant obtenu selon l'expression

$$C(i, j) = E\left(\left(y_i - E(y_i)\right)\left(y_j - E(y_j)\right)\right) \tag{7}$$

où

- E(.) signifie l'espérance mathématique.
- $y_i$ correspond à la $i^{\text{ème}}$ composante du vecteur **y** représentant le signal mesuré **C** correspond à une matrice de covariance de signaux physiologiques lorsque ces derniers correspondent à de l'activité de fond cérébrale, dite « *baseline* », considérée comme du bruit. Le sujet examiné est alors au repos.

A l'étape 145, on établit une matrice de covariance de sources pondérée $\tilde{R}$, en attribuant, à chaque terme $\tilde{R}_{ii}$ de la diagonale dont l'indice correspondant à une zone élémentaire d'intérêt ($i \in$ EM'), une valeur inférieure à celle d'un terme $\tilde{R}_{ii}$ de la diagonale dont l'indice ne correspondant pas à une zone élémentaire d'intérêt ($i \notin$ EM'). Alors que les signaux sources sont reconstruits en $M$ points $P_m$, on améliore ainsi la précision de la reconstruction au niveau des zones élémentaires d'intérêt, ou plus précisément en chaque point associé à une zone élémentaire d'intérêt. On peut ainsi parler de pénalisation spatiale, car chaque point du maillage est affecté d'un terme de pénalisation ($\lambda$ ou $\lambda'$) dépendant de sa position dans le maillage. La matrice de covariance de sources est initialisée selon l'expression suivante :

- $\tilde{R}_{ij} = 0$ si i≠j, les sources élémentaires étant considérées comme indépendantes l'une de l'autre,
-

$$\tilde{R}_{ii} = \lambda' \ \text{si i} \in \text{EM}' \tag{8}$$

- $R_{ij} = \lambda$ si i $\notin$ EM'

avec $\lambda' < \lambda$, et de préférence $\lambda' \leq 10\ \lambda$ voire $\lambda' \leq 100\ \lambda$ et $\lambda'$ étant deux réels strictement positifs.

Cela revient à diminuer, à priori, la part du bruit affectant l'estimation du signal source au niveau des zones élémentaires d'intérêt relativement aux autres zones élémentaires. Ainsi, d'une façon générale la matrice de covariance de sources est initialisée de telle sorte que les termes de sa diagonale, correspondant à des zones élémentaires d'intérêt sont inférieurs aux termes de sa diagonale ne correspondant pas à des zones élémentaires d'intérêt.

A l'étape 140, on estime l'activité électrique dans chaque zone élémentaire du tissu, en résolvant l'équation :

$$\hat{x} = \tilde{R}\, A^T \left( A\tilde{R}A^T + C \right)^{-1} y \tag{9}$$

avec :

- $\tilde{x}$ : vecteur de dimension $M$, chaque terme $\hat{x}$ (i) de ce vecteur correspondant à l'estimation de l'activité électrique au point Pi représentant une zone élémentaire i parmi l'ensemble des $M$ zones élémentaires ;
- $\tilde{R}$ : matrice de covariance de sources (dimension $M \times M$), telle que précédemment définie ;
- **A** : matrice de transmission ou « *leadfield matrix* » ;
- **C** : matrice de covariance de bruit, de dimension ($N \times N$) ;
- **y** : vecteur des mesures des signaux physiologiques, de dimension $N$.

L'homme du métier comprendra que la détermination des matrices **A**, **C** et $\tilde{R}$ ne suit pas nécessairement à l'ordre prévu dans ce premier exemple. Par exemple, la matrice $\tilde{R}$ peut être définie préalablement à la matrice **C** et/ou la matrice **A**.

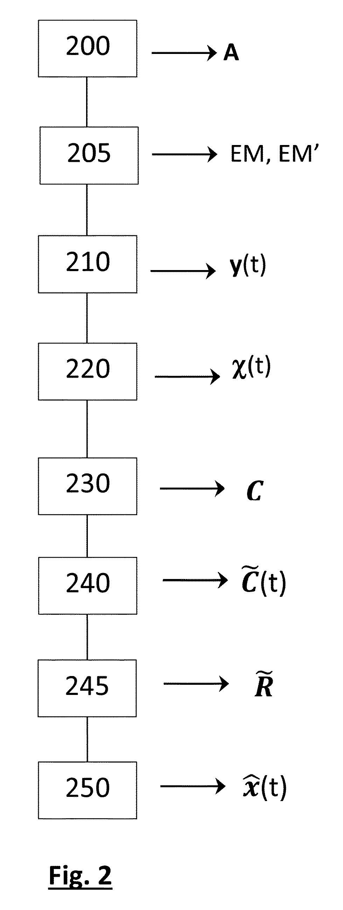

La Fig. 2 représente de manière schématique une méthode d'estimation de l'activité électrique au sein d'un tissu d'un sujet, selon un deuxième mode de réalisation de l'invention. Selon ce mode de réalisation, l'enregistrement des signaux a lieu lorsque le sujet effectue, imagine ou visualise une tâche, cette dernière pouvant être représentée par une variable binaire $\eta(t)$ indicative d'un stimulus sensoriel, par exemple visuel ou auditif. Par exemple, lorsque la variable $\eta(t)$ prend la valeur 1, le stimulus est appliqué et lorsqu'elle prend la valeur 0, il ne l'est pas. Lorsque le stimulus est appliqué, le sujet effectue, imagine ou visualise la tâche en question.

Les étapes 200 et 205 sont respectivement analogues aux étapes 100 et 105 précédemment décrites.

Au cours de l'étape 210, Le stimulus peut être répété de manière à acquérir, une pluralité de séquences $\mathbf{y}(t)$ où **y** est, comme défini précédemment, le vecteur (de dimension $N$) des signaux physiologiques acquis par les différents capteurs au temps $t$).

A l'étape 220, on calcule pour chaque composante $y_n(t)$, $n=1,...,N$, du vecteur $\mathbf{y}(t)$, un coefficient $\chi_n$ représentatif de la corrélation entre le signal $y_n(t)$ et le stimulus $\eta(t)$, sur une plage temporelle donnée, le coefficient $\chi_n$ étant d'autant plus élevé en valeur absolue que la corrélation entre cette composante et le stimulus est importante dans cette plage temporelle. Le coefficient de corrélation $\chi_n$ peut être obtenu selon différentes variantes, comme décrit plus loin. De manière générale, le coefficient $\chi_n$ dépend de la plage temporelle considérée pour le calcul de la corrélation et par conséquent du temps. Pour cette raison, il sera noté par la suite $\chi_n(t)$.

Au terme de la phase d'apprentissage, on dispose ainsi d'une pluralité de coefficients $\chi_n(t)$, $n=1,...,N$, indiquant, en fonction du temps, dans quelle mesure les différents signaux physiologiques sont corrélés « avec le stimulus », autrement dit, sont pertinents vis-à-vis de la tâche en question.

A l'étape 230, on calcule la matrice de covariance du bruit, C. Cette matrice de covariance de bruit est avantageusement obtenue en absence d'application du stimulus et de réalisation de tâche par le sujet, en l'occurrence lorsque $\eta(t) = 0$. Autrement dit, la composante $C_{ij}$ de la matrice de covariance est obtenue par :

$$C_{ij} = E\left[ (y_i - E(y_i))(y_j - E(y_j)) \right] \text{ lorsque } \qquad \eta(t) = 0 \qquad (10)$$

L'espérance mathématique $E(Z)$ peut être estimée à partir de la moyenne de $Z$ sur l'intervalle de temps pendant lequel le stimulus est absent.

Ce mode de réalisation comporte une étape 240, au cours de laquelle on pondère les coefficients (ici les termes diagonaux) de la matrice de covariance de bruit C au moyen des coefficients de corrélation précités, de manière à pénaliser, en termes de rapport signal sur bruit, les signaux physiologiques présentant une faible corrélation avec ce stimulus. Cette pondération favorise corrélativement, en termes de rapport signal sur bruit, les signaux physiologiques présentant une corrélation élevée avec le stimulus. La pénalisation se traduit par une augmentation des coefficients de la matrice de covariance relatifs aux signaux physiologiques faiblement corrélés avec le stimulus, dans la mesure où la matrice de covariance est celle de la covariance de bruit.

Cette pondération est dynamique dans la mesure où les coefficients de pondération varient en fonction du temps. Le résultat de cette pondération est une matrice de covariance pondérée notée $\tilde{\mathbf{C}}(t)$, laquelle évolue dans le temps. Par exemple, les coefficients de la matrice $\tilde{\mathbf{C}}(t)$ pourront être obtenus à partir des coefficients de la matrice de covariance de bruit **C**, de la manière suivante :

$$\tilde{C}_{ij}(t) = C_{ij} \text{ pour } i = 1,...,N, j = 1,...,N, i \neq j$$

$$\tilde{C}_{ii}(t) = \frac{C_{ii}}{1 + \gamma^2 \chi_i^2(t)} \text{ pour } i = 1,...,N \qquad (11)$$

où $\gamma$ est un coefficient prédéterminé. Avantageusement, les coefficients de corrélation $\chi_i(t)$, $i = 1,...,N$, sont normalisés :

$$\chi_i(t) = \frac{\chi_i(t) - \min(\chi_i)}{\max(\chi_i) - \min(\chi_i)} \qquad (12)$$

de sorte qu'ils prennent leurs valeurs dans l'intervalle [0,1]. D'autres fonctions de pondération pourront être envisagées par l'homme du métier sans sortir du cadre de la présente invention.

L'étape 245 est analogue à l'étape 145 du précédent mode de réalisation.

A l'étape, 250, on effectue, à chaque instant, une estimation de l'activité électrique dans le tissu à partir du vecteur des signaux physiologiques **y**, de la matrice **A** de transmission du champ ainsi que de la matrice de covariance de bruit pondérée, **C̃** :

$$\hat{x}(t) = \tilde{R}\, A^T \left( A\tilde{R}A^T + \tilde{C}(t) \right)^{-1} y(t) \qquad (13)$$

L'expression (13) suppose d'inverser une matrice à chaque plage temporelle considérée. En pratique, on pourra se contenter de n'effectuer cette inversion que tous les $N_f$ fenêtres temporelles ($N_f$ étant un entier supérieur à 1) en remplaçant dans l'expression (8) les coefficients de corrélation par leurs moyennes respectives sur $N_f$ fenêtres temporelles.

On notera que selon le premier mode de réalisation, l'estimation de l'activité des sources est réalisée en mettant en oeuvre une matrice de covariance de sources spatialement pondérée, dans le sens ou la pondération est réalisée en fonction de la localisation de chaque source. Dans deuxième mode de réalisation, on combine la matrice de covariance de sources spatialement pondérée à une matrice de covariance de bruit dans l'espace capteurs, cette dernière étant pondérée en fonction de la corrélation temporelle entre les signaux mesurés et la tâche effectuée.

Dans tous les cas, l'activité électrique ainsi estimée peut être représentée sous forme d'image pour localiser l'activité ou bien traitée, dans le cas d'une activité cérébrale, pour générer une commande neuronale directe. Dans ce dernier cas, le traitement en question pourra comprendre l'intégration du module du vecteur $\hat{x}$ sur une zone prédéterminée du cerveau et la comparaison du résultat d'intégration à un seuil, ou encore une corrélation spatiale du vecteur $\hat{x}$ avec un pattern prédéterminé.

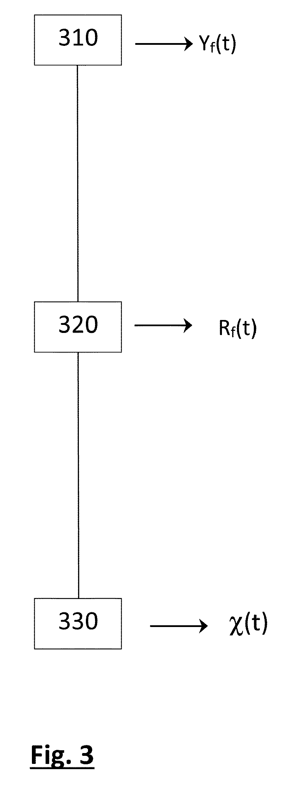

La Fig. 3 représente de manière schématique un exemple de calcul du coefficient de corrélation d'un signal physiologique avec un signal de stimulus, c'est-à-dire, plus précisément, d'un signal $y_n(t)$ fourni par un capteur et le stimulus $\eta(t)$, tel que défini plus haut. Le signal physiologique $y_n(t)$ sera noté dans la suite simplement $y(t)$, le calcul étant identique quel que soit le capteur.

Dans une première étape, 310, on calcule une transformée temps-fréquence ou une transformée temps-échelle du signal physiologique $y(t)$. La transformée temps-fréquence peut être par exemple une transformée de Fourier court-terme par pondération à l'aide d'une fenêtre temporelle glissante, la transformée temps-échelle peut être une transformée par ondelettes ou CWT (*Continuous Wavelet Transform*), de manière connue en soi. On pourra utiliser à cette fin une ondelette de Morlet-Gabor ou une ondelette dite en chapeau mexicain.

Dans tous les cas, on obtient une représentation fréquentielle en fonction du temps, $Y_f(t)$, du signal physiologique $y(t)$, le terme $Y_f(t)$ donnant la composante fréquentielle (ou plus précisément dans une bande fréquentielle) « instantanée » du signal $y(t)$.

Le cas échéant ces composantes fréquentielles peuvent être lissées dans le temps par un filtrage passe-bas, par exemple au moyen d'une moyenne glissante avec coefficient d'oubli.

Dans une seconde étape, 320, on calcule le coefficient de Pearson $R_f$ de chaque composante fréquentielle $Y_f$ de la manière suivante :

$$R_f(t) = \frac{1}{\sigma_\eta . \sigma_{Y_f}} \int\limits_{[t,t+T]} Y_f(u)\left(\eta(u) - \overline{\eta}\right) du \qquad (14)$$

l'intégration sur la fenêtre glissante $[t,t+T]$ pouvant bien entendu être réalisée au moyen d'une sommation discrète et le calcul étant effectué pour un ensemble discret de fréquence. $\sigma_\eta$ et $\sigma_{Y_f}$ représentent respectivement la variance du stimulus $\eta$ et de la composante fréquentielle $Y_f$ et $\overline{\eta}$ est la valeur moyenne de $\eta$ sur la fenêtre glissante en question.

Dans une troisième étape, 330, on calcule le coefficient de corrélation $\chi(t)$ du signal physiologique $y(t)$ avec le stimulus $\eta(t)$ à partir des coefficients de Pearson $R_f(t)$.

Par exemple, on pourra prendre pour $\chi(t)$ la valeur extrémale de $R_f(t)$ dans la plage de fréquence d'intérêt :

$$\chi(t) = \underset{f}{extrem}\left[ R_f(t) \right] \qquad (15)$$

On rappelle que la valeur extrémale d'une fonction est celle de la valeur maximale et de la valeur minimale qui est la plus grande en valeur absolue. De manière équivalente, puisqu'il n'intervient précédemment qu'en valeur absolue,

EP 2 988 224 A1

le coefficient de corrélation pourra être choisi comme le maximum de Figure 8 $|R_f(t)|$ dans la plage de fréquence d'intérêt. D'autres variantes de calcul du coefficient de corrélation pourront être envisagées par l'homme du métier, par exemple l'intégration de $|R_f(t)|$ ou de $(R_f(t))^2$ sur la plage de fréquence d'intérêt.

Quel que soit le mode de réalisation, l'établissement de la matrice de covariance de sources R telle que précédemment décrite permet de simuler, avec une précision accrue, les signaux neuronaux en des points particuliers, et cela préalablement à l'introduction de moyens invasifs, en particulier des électrodes corticales. De telles simulations permettent d'optimiser le placement d'électrodes, en identifiant, par une méthode non invasive (MEG, EEG), les points de la surface corticale optimums du point de vue de l'activité électrique corticale, au regard d'une application visée. Il peut s'agir par exemple de points, au niveau desquels le signal cortical reconstruit présente une bonne corrélation avec une tâche donnée. Il peut également s'agir de points au niveau desquels la réactivité du rythme alpha est particulièrement élevée.

## Revendications

1. Méthode d'estimation de l'activité électrique au sein d'un tissu d'un sujet, dans laquelle

   - on décompose le tissu (100, 200) en une pluralité de zones élémentaires et on détermine une matrice de transition reliant l'activité électrique en chaque zone à un signal physiologique autour du tissu,
   - on réalise l'acquisition (110, 210) d'une pluralité de signaux physiologiques grâce à une pluralité de capteurs disposés autour du tissu,
   - on estime (130, 230) une matrice de covariance de bruit ;
   ladite méthode étant **caractérisée en ce que** :

   - on définit (105, 205) des zones élémentaires d'intérêt parmi les zones élémentaires, le nombre de zones élémentaires d'intérêt étant strictement inférieur au nombre de zones élémentaires
   - on établit (145, 245) une matrice de covariance de sources, de telle sorte que les termes de sa diagonale, correspondant aux zones élémentaires d'intérêt, soient inférieurs aux autres termes de sa diagonale,
   - on estime (150, 250) l'activité électrique, en au moins une zone élémentaire, à partir des signaux physiologiques, de la matrice de covariance de sources et de la matrice de covariance de bruit.

2. Méthode d'estimation de l'activité électrique au sein d'un tissu selon la revendication 1, **caractérisée en ce que** l'estimation est basée sur un critère MNE.

3. Méthode d'estimation de l'activité électrique au sein d'un tissu selon la revendication 2, **caractérisée en ce que** la matrice bruit est calculée comme la matrice de covariance des signaux physiologiques sur une fenêtre temporelle dans laquelle le sujet est au repos.

4. Méthode d'estimation de l'activité électrique au sein d'un tissu selon la revendication 2, **caractérisée en ce que** l'activité électrique dans chaque zone élémentaire est estimée au moyen de :

$$\hat{x} = \tilde{R}\,A^T\big(A\tilde{R}A^T + C\big)^{-1}y$$

où $\hat{x}$ est un vecteur représentant l'activité électrique dans les différentes zones élémentaires, **y** est un vecteur représentant les signaux physiologiques acquis par les capteurs, **A** est une matrice estimant le signal mesuré à chaque capteur pour des sources de puissance unitaire situées dans chaque zones élémentaires, **C** est la matrice de covariance de bruit et **R̃** est la matrice de covariance de sources.

5. Méthode d'estimation de l'activité électrique au sein d'un tissu selon l'une des revendications précédentes, selon laquelle la matrice de covariance de sources est établie au moyen de :

   - $\tilde{R}_{ij} = 0$ si $i \neq j$, les sources élémentaires étant considérée comme indépendante l'une de l'autre,
   - $\tilde{R}_{ii} = \lambda'$, lorsque la zone élémentaire i est une zone élémentaire d'intérêt
   - $\tilde{R}_{ii}\,\lambda$, lorsque la zone élémentaire i n'est pas une zone élémentaire d'intérêt,

λ et λ' étant des réels strictement positifs, avec λ'< λ,,

**6.** Méthode d'estimation de l'activité électrique au sein d'un tissu selon l'une des revendications précédentes, selon laquelle l'activité électrique est associée à une tâche, effectuée, imaginée ou réalisée par le sujet lorsque celui-ci reçoit un stimulus, l'activité électrique étant alors mesurée dans une période temporelle suivant ledit stimulus, la méthode comportant les étapes suivantes :

- on calcule (220) des coefficients de corrélation entre les différents signaux physiologiques et un signal représentatif dudit stimulus
- on pondère (240) les coefficients de la matrice de covariance des signaux physiologiques, à l'aide des coefficients de corrélation, de sorte à pénaliser, en termes de rapport signal sur bruit, les signaux physiologiques qui sont faiblement corrélés avec le stimulus, la pénalisation diminuant les termes de la matrice de corrélation relatifs aux signaux faiblement corrélés avec le stimulus.

**7.** Méthode d'estimation de l'activité électrique au sein d'un tissu selon la revendication 6, selon laquelle l'estimation est basée sur un critère de type MNE, **caractérisée en ce que** la matrice de covariance des signaux physiologiques est une matrice de covariance de bruit, calculée sur une fenêtre temporelle où le stimulus est absent, et que l'activité électrique dans chaque zone élémentaire est estimée au moyen de :

$$\widehat{x}(t) = \widetilde{R}\, A^T \left( A\widetilde{R}A^T + \widetilde{C}(t) \right)^{-1} y(t)$$

où $\widehat{x}(t)$ est un vecteur représentant l'activité électrique au temps t dans les différentes zones élémentaires, $y(t)$ est un vecteur représentant les signaux physiologiques acquis par les capteurs au temps t, **A** est une matrice estimant le signal mesuré à chaque capteur pour des sources de puissance unitaire situées dans chaque zone élémentaire, $\widetilde{C}(t)$ est la matrice de covariance de bruit au temps t et $\widetilde{R}$ est la matrice de covariance de sources.

**8.** Méthode d'estimation de l'activité électrique au sein d'un tissu selon la revendication 7, **caractérisée en ce que** les coefficients de matrice de covariance de bruit pondérée sont obtenus à partir de la matrice de covariance de bruit au moyen de la relation suivante :

$$\tilde{C}_{ij}(t) = C_{ij} \text{ pour } i=1,...,N, j=1,...,N, i \neq j$$

$$\tilde{C}_{ii}(t) = \frac{C_{ii}}{1+\gamma^2\chi_i^2(t)} \text{ pour } i=1,...,N$$

où les coefficients $\tilde{C}_{ij}(t)$, $i$=1,...,$N$, $j$=1,...,$N$, sont les coefficients de la matrice de covariance de bruit pondérée, les coefficients $C_{ij}$, $i$=1,...,$N$,$j$=1,...,$N$ sont les coefficients de la matrice de covariance de bruit, $N$ est le nombre de capteurs, $\gamma$ est une constante réelle prédéterminé et $\chi_i(t)$, $i$=1,...,$N$, sont les coefficients de corrélation des signaux physiologiques acquis par les différents capteurs avec le signal représentatif du stimulus.

**9.** Méthode d'estimation de l'activité électrique au sein d'un tissu selon la revendication 8, **caractérisée en ce que** les coefficients de corrélation $\chi_i(t)$, $i$=1,...,$N$ font l'objet d'une normalisation préalablement à la pondération des coefficients de la matrice de covariance de bruit.

**10.** Méthode d'estimation de l'activité électrique au sein d'un tissu selon l'une des revendications 7 à 9, **caractérisée en ce que** les coefficients de corrélation sont obtenus en effectuant (310) une transformée temps-fréquence ou temps-échelle de chaque signal physiologique pour obtenir une pluralité de composantes fréquentielles ($Y_f(t)$) de ce signal en fonction du temps, en calculant (320) les coefficients de Pearson ($R_f(t)$) entre lesdites composantes fréquentielles et le signal représentatif du stimulus, le coefficient de corrélation ($\chi(t)$) relatif à un signal physiologique étant déterminé à partir desdits coefficients de Pearson obtenus pour ce signal.

**11.** Méthode d'estimation de l'activité électrique au sein d'un tissu selon la revendication 10, **caractérisée en ce que** le coefficient de corrélation ($\chi(t)$ relatif à un signal physiologique est obtenu (330) comme la valeur extrémale des coefficients de Pearson pour les différentes composantes fréquentielles de ce signal.

| | |
|---|---|
| 100 | ⟶ **A** |
| 105 | ⟶ EM, EM' |
| 110 | ⟶ **y** |
| 130 | ⟶ **C** |
| 145 | ⟶ $\widetilde{R}$ |
| 150 | ⟶ $\widehat{x}$ |

## Fig. 1

| | |
|---|---|
| 200 | $\longrightarrow$ A |
| 205 | $\longrightarrow$ EM, EM' |
| 210 | $\longrightarrow$ y(t) |
| 220 | $\longrightarrow$ $\chi$(t) |
| 230 | $\longrightarrow$ $C$ |
| 240 | $\longrightarrow$ $\widetilde{C}$(t) |
| 245 | $\longrightarrow$ $\widetilde{R}$ |
| 250 | $\longrightarrow$ $\widehat{x}$(t) |

**Fig. 2**

$$310 \longrightarrow Y_f(t)$$

$$320 \longrightarrow R_f(t)$$

$$330 \longrightarrow \chi(t)$$

**Fig. 3**

Europäisches
Patentamt

European
Patent Office

Office européen
des brevets

## RAPPORT DE RECHERCHE EUROPEENNE

Numéro de la demande

EP 15 18 1681

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | ARTHUR K LIU ET AL: "Spatiotemporal imaging of human brain activity using functional MRI constrained magnetoencephalography data: Monte Carlo simulations", PROCEEDINGS OF THE NATIONAL ACADEMY OF SCIENCES OF THE UNITED STATES OF AMERICA NEUROBIOLOGY, vol. 95, no. 6, juillet 1998 (1998-07), pages 8945-8950, XP055179344, | 1-5 | INV. G06F17/18 G06F3/01 A61B5/04 |
| Y | * page 8946 - page 8949 * ----- | 6-11 | |
| Y | EP 2 679 151 A1 (COMMISSARIAT ENERGIE ATOMIQUE [FR]) 1 janvier 2014 (2014-01-01) | 6-11 | |
| A | * page 4 - page 6 * ----- | 1-5 | |
| A | CN 103 699 226 A (UNIV TIANJIN) 2 avril 2014 (2014-04-02) * le document en entier * ----- | 1-11 | |
| A | MOLINS A ET AL: "Quantification of the benefit from integrating MEG and EEG data in minimum @?2-norm estimation", NEUROIMAGE, ACADEMIC PRESS, ORLANDO, FL, US, vol. 42, no. 3, septembre 2008 (2008-09), pages 1069-1077, XP026446469, ISSN: 1053-8119 [extrait le 2008-06-14] * le document en entier * ----- | 1-11 | DOMAINES TECHNIQUES RECHERCHES (IPC) A61B G06F |

Le présent rapport a été établi pour toutes les revendications

| Lieu de la recherche | Date d'achèvement de la recherche | Examinateur |
|---|---|---|
| Berlin | 17 décembre 2015 | Huguet Serra, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

# EP 2 988 224 A1

## ANNEXE AU RAPPORT DE RECHERCHE EUROPEENNE
## RELATIF A LA DEMANDE DE BREVET EUROPEEN NO.

EP 15 18 1681

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-12-2015

| Document brevet cité au rapport de recherche | | Date de publication | Membre(s) de la famille de brevet(s) | | Date de publication |
|---|---|---|---|---|---|
| EP 2679151 | A1 | 01-01-2014 | EP FR US | 2679151 A1<br>2992543 A1<br>2014107464 A1 | 01-01-2014<br>03-01-2014<br>17-04-2014 |
| CN 103699226 | A | 02-04-2014 | AUCUN | | |

Pour tout renseignement concernant cette annexe : voir Journal Officiel de l'Office européen des brevets, No.12/82

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Beamforming and its application to brain connectivity. **A. FUCHS ; V.K. JIRSA ; R.A. MCLNTOSH.** Handbook of Brain Connectivity. Springer Verlag, 2007, 357-378
- **O. HAUK et al.** Comparison of noise-normalized minimum norm estimates of MEG analysis using multiple resolution metrics. *Neuroimage,* 2011, vol. 54, 1966-1974
- **MONTE CARLO.** *Proceedings of the National Academy of Sciences of the United States of America,* Juillet 1998, vol. 95 (15), 8945-8950
- **J. VRBA et al.** Signal processing in magnetoencephalography. *Methods,* 2001, vol. 25, 249-271